# EUROPÄISCHE PATENTSCHRIFT

(11) **EP 3 346 273 B1**
(45) Veröffentlichungstag und Bekanntmachung des Hinweises auf die Patenterteilung: **04.01.2023**
(21) Anmeldenummer: 17172220.0
(22) Anmeldetag: 13.09.2013
(51) Int. Cl.: G01N 33/68, C07K 7/04

(54) **NEUE, VON D3 ABGELEITETE D-ENANTIOMERE PEPTIDE UND DEREN VERWENDUNG**
NOVEL D-ENANTIOMERIC PEPTIDES DERIVED FROM D3 AND THEIR USE
NOUVEAU PEPTIDE D'ÉNANTIOMÈRE DÉRIVÉ DE D3 ET SON UTILISATION

(30) Priorität: 14.09.2012 DE 102012108598
(43) Veröffentlichungstag der Anmeldung: 11.07.2018
(62) Teilanmeldung aus: 13776426.2
(73) Patentinhaber: Priavoid GmbH, 40225 Düsseldorf (DE)
(72) Erfinder: Willbold, Dieter, 52425 Jülich (DE); Brener, Oleksander, 40591 Düsseldorf (DE); Funke, Susanne Aileen, 96242 Sonnefeld (DE); Nagel-Steger, Luitgard, 40764 Langenfeld (DE); Klein, Antonia Nicole, 52353 Düren (DE); Bartnik, Dirk, 41334 Nettetal (DE)
(74) Vertreter: Fitzner, Uwe

(56) Entgegenhaltungen:
- WO-A2-02/42462
- WO-A2-2006/005706
- WO-A2-2007/047967
- WO-A2-2010/062570
- WO-A2-2011/147797
- US-A1- 2005 158 306
- RANJINI K. SUNDARAM ET AL: "Novel Detox Gel Depot Sequesters &bgr;-Amyloid Peptides in a Mouse Model of Alzheimer's Disease", INTERNATIONAL JOURNAL OF PEPTIDE RESEARCH AND THERAPEUTICS, Bd. 18, Nr. 2, 1. Juni 2012 (2012-06-01), Seiten 99-106, XP055070073, ISSN: 1573-3149, DOI: 10.1007/s10989-011-9283-7
- ANDREAS MÜLLER-SCHIFFMANN ET AL: "Combining Independent Drug Classes into Superior, Synergistically Acting Hybrid Molecules", ANGEWANDTE CHEMIE INTERNATIONAL EDITION, Bd. 49, Nr. 46, 8. November 2010 (2010-11-08), Seiten 8743-8746, XP055083717, ISSN: 1433-7851, DOI: 10.1002/anie.201004437
- DIRK BARTNIK ET AL: "Differently selected D-enantiomeric peptides act on different A.beta. species", REJUVENATION RESEARCH, MARY ANN LIEBERT, NEW ROCHELLE, NY, US, Bd. 13, Nr. 2-3, 12. Mai 2010 (2010-05-12) , Seiten 202-205, XP002654152, ISSN: 1549-1684, DOI: 10.1089/REJ.2009.0924 [gefunden am 2009-12-02]
- ZHANG G ET AL: "Multiple-Peptide Conjugates for Binding ß-Amyloid Plaques of Alzheimer's Disease", BIOCONJUGATE CHEMISTRY,, Bd. 14, 1. Januar 2003 (2003-01-01), Seiten 86-92, XP002458160, ISSN: 1043-1802, DOI: 10.1021/BC025526I
- KATJA WIESEHAN ET AL: "Selection of D-Amino-Acid Peptides That Bind to Alzheimer's Disease Amyloid Peptide A[beta]142 by Mirror Image Phage Display", CHEMBIOCHEM, Bd. 4, Nr. 8, 4. August 2003 (2003-08-04), Seiten 748-753, XP055069247, ISSN: 1439-4227, DOI: 10.1002/cbic.200300631
- SIDHARTHA M. CHAFEKAR ET AL: "Branched KLVFF Tetramers Strongly Potentiate Inhibition of &bgr;-Amyloid Aggregation", CHEMBIOCHEM, Bd. 8, Nr. 15, 15. Oktober 2007 (2007-10-15), Seiten 1857-1864, XP055052606, ISSN: 1439-4227, DOI: 10.1002/cbic.200700338
- STAINS CLIFF I ET AL: "Molecules that target beta-amyloid", CHEMMEDCHEM, WILEY-VCH, Bd. 2, Nr. 12, 1. Dezember 2007 (2007-12-01), Seiten 1675-1692, XP009107501, ISSN: 1860-7179, DOI: 10.1002/CMDC.200700140
- SUSANNE AILEEN FUNKE ET AL: "Mirror image phage display-a method to generate d-peptide ligands for use in diagnostic or therapeutical applications", MOLECULAR BIOSYSTEMS, Bd. 5, Nr. 8, 1. Januar 2009 (2009-01-01), Seite 783, XP055069250, ISSN: 1742-206X, DOI: 10.1039/b904138a
- THOMAS VAN GROEN ET AL: "Reduction of Alzheimer's Disease Amyloid Plaque Load in Transgenic Mice by D3, a D-Enantiomeric Peptide Identified by Mirror Image Phage Display", CHEMMEDCHEM, Bd. 3, Nr. 12, 15. Dezember 2008 (2008-12-15), Seiten 1848-1852, XP055239322, ISSN: 1860-7179, DOI: 10.1002/cmdc.200800273
- SUSANNE AILEEN FUNKE ET AL: "-Enantiomeric Peptide D3 Improves the Pathology and Behavior of Alzheimer's Disease Transgenic Mice", ACS CHEMICAL NEUROSCIENCE, Bd. 1, Nr. 9, 15. September 2010 (2010-09-15), Seiten 639-648, XP055239332, US ISSN: 1948-7193, DOI: 10.1021/cn100057j

## Beschreibung

Die vorliegende Erfindung betrifft ein Peptid, enthaltend mindestens eine Aminosäuresequenz gemäß SEQ ID NO: 2 und dessen Verwendung, insbesondere in der Medizin.

Aufgrund der demographischen Entwicklung in den nächsten Jahrzehnten wird sich die Zahl der Personen, die an altersbedingten Erkrankungen leiden, erhöhen. Hier ist insbesondere die sog. Alzheimer-Krankheit (AD, Alzheimersche Demenz, lateinisch = Morbus Alzheimer) zu nennen.

Ein Merkmal der Alzheimer-Erkrankung sind extrazelluläre Ablagerungen des Amyloid-Beta-Peptids (A-Beta-Peptid). Diese Ablagerung des A-Beta-Peptids in Plaques ist typischerweise in den Gehirnen von AD-Patienten post mortem festzustellen. Deshalb werden verschiedene Formen des A-Beta-Peptids - wie z.B. Fibrillen - für die Entstehung und das Fortschreiten der Krankheit verantwortlich gemacht. Zusätzlich werden seit einigen Jahren die kleinen, frei diffundierbaren A-Beta-Oligomere als hauptsächliche Verursacher der Entstehung und des Fortschritts der AD gesehen.

A-Beta-Monomere, als Bausteine der A-Beta-Oligomere, entstehen im menschlichen Körper ständig und sind vermutlich per se nicht toxisch. Möglicherweise haben sie sogar eine neuroprotektive Funktion. A-Beta-Monomere können sich in Abhängigkeit von ihrer Konzentration zufällig zusammen lagern. Die Konzentration ist abhängig von ihrer Bildungs- und Abbaurate im Körper. Findet mit zunehmendem Alter eine Erhöhung der Konzentration an A-Beta-Monomeren im Körper statt, so ist eine spontane Zusammenlagerung der Monomere zu A-Beta-Oligomeren immer wahrscheinlicher. Die so entstandenen A-Beta-Oligomere könnten sich analog zu den Prionen vermehren und letztendlich zur Morbus Alzheimer-Krankheit führen.

Bisher existiert kein Wirkstoff oder Medikament, das gegen die Ursachen von AD wirkt. Die bisher eingesetzten und zugelassenen Medikamente mildern einige der bei der AD auftretenden Symptome. Sie sind aber nicht in der Lage, den Krankheitsfortschritt zu verlangsamen oder eine Heilung herbeizuführen. Es existieren einige Substanzen, die im Tierversuch Erfolge bei der Prävention, aber nicht (unbedingt) bei der Behandlung von AD gezeigt haben.

Ein wichtiger Unterschied zwischen Prävention und Behandlung oder gar Heilung der AD liegt in der Tatsache, dass eine Prävention möglicherweise schon durch die Verhinderung der Bildung der ersten A-Beta-Oligomere erreicht werden kann. Zur Prävention sind einige, wenige A-Beta-Liganden ausreichend, die nicht notwendigerweise hochaffin und selektiv bezüglich der A-Beta-Oligomere sind.

Die Bildung der A-Beta-Oligomere aus vielen Monomeren ist eine Reaktion hoher Ordnung und damit in hoher Potenz von der A-Beta-Monomer-Konzentration abhängig. Somit führt schon eine kleine Verringerung der aktiven A-Beta-Monomer-Konzentration zu einer Verhinderung der Bildung der ersten A-Beta-Oligomere. Auf diesem Mechanismus basieren die bisher sich in der Entwicklung befindlichen eher präventiven Therapiekonzepte und Substanzen. Bei der Behandlung von AD ist jedoch von einer völlig veränderten Situation auszugehen. Hier liegen A-Beta-Oligomere oder evtl. auch schon größere Polymere oder Fibrillen vor, die sich durch Prion-ähnliche Mechanismen vermehren. Diese Vermehrung ist jedoch eine Reaktion niederer Ordnung und ist somit kaum noch von der A-Beta-Monomer-Konzentration abhängig.

Die aus dem Stand der Technik bekannten Substanzen verringern die Konzentration von A-Beta-Monomeren und/oder Oligomeren auf verschiedenste Art und Weise. So sind z.B. Gamma-Sekretase-Modulatoren bekannt, die im Tierversuch zur Prävention eingesetzt wurden.

Aus der WO 02/081505 sind verschiedene Sequenzen von D-Aminosäuren bekannt, die an A-Beta-Peptide binden. Diese Sequenzen aus D-Aminosäuren binden mit einer Dissoziationskonstante (K_{D}-Wert) von 4 µmol an Amyloid-Beta-Peptide.

Aus der WO 2011/147797 sind Hybridverbindungen bekannt, bestehend aus Aminopyrazolen und Peptiden, die A-Beta-Oligomerisation verhindern.

Bei vielen Substanzen, die im Tierversuch positive Ergebnisse gezeigt haben, konnte diese Wirkung in klinischen Studien am Menschen nicht bestätigt werden. In klinischen Studien der Phase II und III dürfen nur Menschen behandelt werden, die eindeutig mit AD diagnostiziert wurden. Hier reicht eine geringe Verringerung der A-Beta-Monomerkonzentration nicht mehr aus, um zu verhindern, dass sich aus den bereits vorhandenen A-Beta-Oligomeren noch mehr bilden, z.B. durch einen Prion-ähnlichen Mechanismus. Die Vermehrung der A-Beta-Oligomere oder noch besser ihre Zerstörung oder Unschädlichmachung ist aber unbedingt erforderlich, um Einfluss auf den Krankheitsverlauf zu nehmen.

Bisher wird die AD hauptsächlich durch neuropsychologische Tests diagnostiziert, durch Untersuchungen an Personen, bei denen bereits Demenzsymptome aufgetreten sind. Es ist jedoch bekannt, dass A-Beta-Oligomere und die zeitlich im Krankheitsverlauf darauf folgenden Fibrillen und Plaques bis zu 20 Jahre vor dem Auftreten der Symptome im Gehirn der Patienten entstehen, und schon irreversible Schäden verursacht haben können. Allerdings gibt es bisher in der Praxis noch keine Möglichkeit, die AD vor dem Ausbruch der Symptome zu diagnostizieren.

Es besteht somit weiterhin ein Bedarf an neuen Verbindungen (Wirkstoffen), die sehr spezifisch und mit hoher Affinität an A-Beta-Oligomere binden, und so deren Vermehrung verhindern. Diese Verbindungen sollten keine unerwünschten Nebenwirkungen zeigen, insbesondere keine Immunreaktion hervorrufen. Die Verbindungen sollen außerdem toxische A-Beta-Oligomere und damit auch die kleinen frei diffundierbaren Oligomere in kleinen Konzentrationen erkennen, vollständig vernichten und/oder deren (Prionenähnliche) Vermehrung verhindern.

Des Weiteren besteht auch ein Bedarf an neuen Verbindungen, die als Sonden für die Erkennung und Markierung von A-Beta-Oligomeren einsetzbar sind, insbesondere, wenn die Krankheit noch nicht weit fortgeschritten ist und die Oligomere erst in geringen Konzentrationen auftreten.

Weitere Aufgabe der vorliegenden Erfindung war es, Substanzen zur Verfügung zu stellen, die nicht nur auf extrazelluläre A-Beta-Peptide fokussieren, wie die meisten aus dem Stand der Technik bekannten Verbindungen, sondern gezielt lösliche A-Beta-Oligomere binden. Ferner sollen die neuen Verbindungen die Fibrillenbildung von A-Beta-Peptiden hemmen oder verhindern.

Aufgabe war es außerdem neue Peptide zur Verfügung zu stellen, bevorzugt Derivate des D-enantiomeren D-Peptids D3 (SEQ ID NO: 11), die im Vergleich zu D3 effizientere Eigenschaften besitzen. Die Eigenschaften sind u.a. Bindungsaffinität und -spezifität für A-Beta-Spezies, Inhibition der A-Beta-Fibrillenbildung, Inhibierung der A-Beta-Zytotoxizität, Präzipitation von A-Beta-Oligomeren, Umwandlung von A-Beta-Fibrillen in nicht-toxische nichtamyloidogene Spezies.

Gelöst wird diese Aufgabe durch ein Peptid enthaltend eine Aminosäuresequenz gemäß SEQ ID NO: 2 (RD2).

Ferner offenbart sind die Peptide SEQ ID NO: 1 (D3-Delta-HTH), SEQ ID NO: 3 (RD 1), SEQ ID NO: 4 (RD3), SEQ ID NO: 5 (DB3), SEQ ID NO: 6 (NT-D3), SEQ ID NO: 7 (DB1), SEQ ID NO: 8 (DB2), SEQ ID NO: 9 (DB4) und/oder SEQ ID NO. 10 (DB5) und/oder Homologe, Fragmente und Teile davon. Gelöst wird diese Aufgabe auch durch Polymere der SEQ ID NO: 2 (RD2).

Ferner offenbart sind auch Polymere der SEQ ID NO: 1 (D3-Delta-HTH), SEQ ID NO: 3 (RD 1), SEQ ID NO: 4 (RD3), SEQ ID NO: 5 (DB3), SEQ ID NO: 6 (NT-D3), SEQ ID NO: 7 (DB1), SEQ ID NO: 8 (DB2), SEQ ID NO: 9 (DB4) und/oder SEQ ID NO. 10 (DB5) und/oder deren Homologe.

Fragmente und Teile können eine ähnliche beziehungsweise identische Wirkung wie die Peptide zeigen.

Die Peptide gemäß SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 und/oder SEQ ID NO. 10 und deren Homologe bestehen zu 100% aus D-Aminosäuren.

Ein Polymer im Sinne der vorliegenden Offenbarung ist gebildet aus 2, 3, 4, 5, 6, 7, 8, 9, 10, 11, 12, 13, 14, 15, 16, 17, 18, 19, 20 oder mehr Monomeren ausgewählt aus der Gruppe bestehend aus SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 und/oder SEQ ID NO. 10 und deren Homologe, die für sich bereits A-Beta-Oligomer.

Erfindungsgemäß können die Polymere aus identischen Monomeren aufgebaut sein oder aus einer Kombination von 2, 3, 4, 5, 6, 7, 8, 9, 10 unterschiedlichen, verschiedenen der oben genannten Monomeren, als sogenannte Kombinations-Polymere Die Monomere können auch teilweise identisch sein. Die Anzahl der identischen Monomeren in den Kombinations-Polymeren ist frei wählbar.

In einer Alternative enthalten die Kombinations-Polymere neben einem Peptid mit der Aminosäuresequenz gemäß SEQ ID NO: 2 mindestens ein Monomer ausgewählt aus der Gruppe bestehend aus SEQ ID NO: 1, , SEQ ID NO: 3, SEQ ID NO: 4, SEQ ID NO: 5, SEQ ID NO: 6, SEQ ID NO: 7, SEQ ID NO: 8, SEQ ID NO: 9 und/oder SEQ ID NO. 10.

Polymere können beispielsweise über chemische Synthese bzw. Peptidsynthese hergestellt werden.

In einer Ausführung der Erfindung sind die Monomere kovalent miteinander verknüpft. In einer weiteren Ausführung sind die Monomere nicht kovalent miteinander verbunden.

Eine kovalente Verbindung bzw. Verknüpfung der Monomer-Einheiten liegt im Sinne der Erfindung vor, falls die Peptide Kopf an Kopf, Schwanz an Schwanz oder Kopf an Kopf linear miteinander verknüpft werden, ohne dass dazwischen Linker oder Linker-Gruppen eingesetzt werden.

Eine nicht kovalente Verknüpfung im Sinne der Erfindung liegt vor, falls die Monomere über Biotin und Streptavidin, insbesondere Streptavidin-Tetramer miteinander verknüpft sind.

In einer Variante der vorliegenden Erfindung können die Monomere linear miteinander verknüpft sein, insbesondere wie oben beschrieben. In einer anderen Variante sind die Monomere verzweigt miteinander zu dem erfindungsgemäßen Polymer verknüpft.

Bei einem verzweigten Polymer kann es sich erfindungsgemäß um ein Dendrimer handeln, bei dem die Monomere kovalent oder nicht kovalent miteinander verknüpft sind.

Alternativ können die Monomere auch mit einem Plattform-Molekül (wie z.B. PEG oder Zucker) verknüpft sein und so ein verzweigtes Polymer bilden.

Alternativ sind auch Kombinationen dieser Optionen möglich.

Monomere und Polymere werden im Folgenden als erfindungsgemäße Peptide bezeichnet.

In einer nicht erfindungsgemäßen Variante handelt es sich um ein Peptid mit der Aminosäuresequenz gemäß SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 und/oder SEQ ID NO. 10 und/oder Homologe davon mit einer Identität von 50%.

"Homologe Sequenzen" oder "Homologe" bedeutet, dass eine Aminosäuresequenz eine Identität mit einer der oben genannten Aminosäuresequenz der Monomere von mindestens 50, 55, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 ,100 % aufweist. Anstelle des Begriffs "Identität" werden in der vorliegenden Beschreibung die Begriffe "homolog" oder "Homologie" gleichbedeutend verwendet. Die Identität zwischen zwei Nukleinsäuresequenzen oder Polypeptidsequenzen wird durch Vergleich mit Hilfe des Programms BESTFIT basierend auf dem Algorithmus von Smith, T.F. und Waterman, M.S (Adv. Appl. Math. 2: 482-489 (1981)) berechnet unter Einstellung folgender Parameter für Aminosäuren: Gap creation penalty: 8 und Gap extension penalty: 2; und folgender Parameter für Nukleinsäuren: Gap creation penalty: 50 und Gap extension penalty: 3. Bevorzugt wird die Identität zwischen zwei Nukleinsäuresequenzen oder Polypeptidsequenzen durch die Identität der Nukleinsäuresequenz / Polypeptidequenz über die jeweils gesamte Sequenzlänge definiert, wie sie durch Vergleich mit Hilfe des Programms GAP basierend auf dem Algorithmus von Needleman, S.B. und Wunsch, C.D. (J. Mol. Biol. 48: 443-453) unter Einstellung folgender Parameter für Aminosäuren berechnet wird: Gap creation penalty: 8 und Gap extension penalty: 2; und die folgenden Parameter für Nukleinsäuren Gap creation penalty: 50 und Gap extension penalty: 3.

Zwei Aminosäuresequenzen sind im Sinne der vorliegenden Erfindung identisch wenn sie dieselbe Aminosäuresequenz besitzen.

Unter Homologe sind in einer nicht erfindungsgemäßen Variante die entsprechenden retro-inversen Sequenzen der oben genannten Monomere zu verstehen. Mit dem Begriff "retro-inverse Sequenz" wird erfindungsgemäß eine Aminosäuresequenz bezeichnet, die sich aus Aminosäuren in der enantiomeren Form zusammensetzt (invers: Chiralität des alpha-C-Atoms invertiert) und bei der zusätzlich die Sequenzreihenfolge zur ursprünglichen Aminosäuresequenz umgekehrt wurde (retro = rückwärts).

In einer weiteren Variante binden die erfindungsgemäßen Peptide an Teile des Amyloid Beta-Peptids.

In einer weiteren nicht erfindungsgemäßen Variante weisen die Peptide Sequenzen auf, die sich von den angegebenen Sequenzen um bis zu drei Aminosäuren unterscheiden.

Ferner können als Peptide auch Sequenzen eingesetzt werden, die die oben genannten Sequenzen enthalten.

In einer weiteren nicht erfindungsgemäßen Variante weisen die Peptide Fragmente der oben genannten Sequenzen auf oder weisen homologe Sequenzen zu den oben genannten Sequenzen auf.

Erfindungsgemäß handelt es sich um ein Peptid zur Verwendung in der Medizin, bevorzugt zur Behandlung der Morbus Alzheimer.

Gemäß der vorliegenden Erfindung besteht das Peptid aus D-Aminosäuren.

In einer weiteren Variante handelt es sich um ein erfindungsgemäßes Peptid zur Verwendung in vivo zur Hemmung der Fibrillenbildung von Amyloid-Beta-Peptiden. Die erfindungsgemäßen Polymere enttoxifizieren die A-Beta-Oligomere oder daraus gebildete Polymere, sowie Fibrillen, indem sie daran binden und so in nicht toxische Verbindungen überführen. Demgemäß ist Gegenstand der vorliegenden Erfindung auch ein Peptide zur Verwendung in vivo zur Enttoxifizierung der A-Beta-Oligomeren, daraus gebildeten Polymeren oder Fibrillen.

Gegenstand der Erfindung sind in einer Ausführung auch erfindungsgemäße Peptide die mit einer weiteren Substanz verknüpft sind.

Bei der Verknüpfung handelt es sich im Sinne der Erfindung um eine chemische Bindung wie sie in Römpp Chemie Lexikon, 9. Auflage, Band 1, Seite 650 ff, Georg Thieme Verlag Stuttgart definiert ist, bevorzugt um eine Hauptvalenz Bindung, insbesondere eine kovalente Bindung.

Bei den Substanzen handelt es sich in einer Variante um Arzneimittel oder Wirkstoffe, definiert gemäß Arzneimittelgesetz §2 beziehungsweise. §4 (19), Stand September 2012. In einer Alternative sind Wirkstoffe therapeutisch aktive Stoffe die als arzneilich wirksame Stoffe verwendet werden. Bevorzugt werden Entzündungshemmer eingesetzt.

Bei den Substanzen handelt es sich in einer weiteren Variante um Verbindungen die die Wirkung der Peptide verstärken.

In einer Alternative sind solche Verbindungen Aminopyrazol und/oder Aminopyrazolderivate. Aminopyrazolderivate im Sinne der Erfindung ist 3-Aminopyrazol-5-carbonsäure oder 3-Nitropyrazol-5-carbonsäure sowie alle Abkömmlinge, in denen die heterocyclische CH-Gruppe gegen -CR- oder -N- oder -O- oder -S- ausgetauscht wurde, sowie alle daraus abgeleiteten peptidischen Dimere, Trimere oder -Tetramere, bevorzugt Aminopyrazol-Trimer.

In einer weiteren Alternative handelt es sich um Verbindungen die die Löslichkeit der Peptide und/oder die Passage der Blut-Hirn-Schranke verbessern.

In einer Alternative haben die Peptide erfindungsgemäß jede beliebige Kombination von mindestens zwei oder mehr Merkmalen der oben beschriebenen Varianten, Ausführungen und/oder Alternativen.

Gegenstand der Erfindung ist auch ein erfindungsgemäßes Peptid zur Verwendung in vivo zur Bindung an aggregierte A-Beta-Peptide.

Ferner offenbart ist ein nicht erfindungsgemäßes Verfahren zur Herstellung des erfindungsgemäßen Peptids durch Peptidsynthese, wie zum Beispiel dem Fachmann bekannt, organische Synthesemethoden für beliebige Verbindungen mit geringem Molekulargewicht (low-molecular weight compounds) und/oder Mutagenese und rekombinante Herstellung.

Die Erfindung betrifft ferner auch eine Zusammensetzung enthaltend das erfindungsgemäße Peptid, insbesondere zur Verwendung in der Behandlung von Morbus Alzheimer.

Gegenstand der vorliegenden Erfindung ist ferner eine Zusammensetzung enthaltend das erfindungsgemäße Peptid, zur Verwendung in vivo zur Verhinderung von toxischen A-Beta-Oligomeren, oder zur Zerstörung von daraus gebildeten Polymere oder Fibrillen.

Die erfindungsgemäße "Zusammensetzung" kann z. B. ein Impfstoff, ein Medikament (z. B. in Tablettenform), eine Injektionslösung, ein Nahrungs- oder Nahrungsergänzungsmittel sein, enthaltend das erfindungsgemäße Peptid in einer aufgrund des Fachwissens herzustellenden Formulierung.

Die Erfindung betrifft ferner auch ein KIT enthaltend das erfindungsgemäße Peptid.

In einem solchen KIT können die erfindungsgemäßen Peptide in Behältern ggf. mit/in Puffern oder Lösungen verpackt sein. Alle Komponenten des KITs können in demselben Behälter oder getrennt voneinander verpackt sein. Ferner kann das KIT Anweisungen für dessen Gebrauch enthalten. Ein solches KIT kann beispielsweise die erfindungsgemäßen in einer Injektionsflasche mit Stopfen und/oder Septum enthalten. Ferner kann darin beispielsweise auch eine Einmal-Spritze enthalten sein.

Ein weiterer Gegenstand der vorliegenden Erfindung ist die Verwendung des erfindungsgemäßen Peptids in vitro als Sonde zur Identifizierung, qualitativen und/oder quantitativen Bestimmung von Amyloid-Beta-Oligomeren oder Fibrillen.

Ferner offenbart ist auch eine nicht erfindungsgemäße Sonde, enthaltend das erfindungsgemäße Peptid zur Identifizierung, qualitativen und/oder quantitativen Bestimmung von Amyloid-Beta-Oligomeren.

Solche Sonden sind von großer Bedeutung, da damit eine frühe Diagnose der AD möglich wird. Mit der Frühdiagnose kann der Krankheit schon in einem sehr frühen Stadium entgegengewirkt werden.

Solche molekularen Sonden können das erfindungsgemäße Polymer enthalten und gegebenenfalls Farbstoffe, Fluoreszenzfarbstoffe, radioaktive Isotope, (PET etc.), Gadolinium (MRI), sowie alternative Stoffe geeignet für die Bildgebung der Sonden und können den Patienten z.B. intravenös injiziert werden. Nach Passage über die Bluthirnschranke können die Sonden an A-Beta-Oligomere und/oder Plaques binden. Die so markierten A-Beta-Oligomere und/oder Plaques können mittels bildgebender Verfahren wie z.B. SPECT, PET, CT, MRT, Protonen-MR-Spektroskopie usw. sichtbar gemacht werden.

Ferner betrifft die Erfindung auch die Verwendung des Peptids zur Verhinderung von Amyloid-Beta-Oligomeren und/oder Amyloid-Beta-Peptid-Aggregaten und/oder Amyloid-Beta-Fibrillen.

Das erfindungsgemäße Peptid wird auch in der Enttoxifizierung von toxischen Amyloid-Beta-Oligomeren und/oder Aggregaten verwendet. Insbesondere wird es verwendet, um an Amyloid-Beta-Oligomere und/oder Aggregate zu binden und so amorphe, nicht toxische Aggregate zu bilden.

Die erfindungsgemäßen Peptide binden besonders gut an A-Beta-Oligomere, insbesondere an lösliche A-Beta-Oligomere.

Eine besonders starke Bindung der erfindungsgemäßen Peptide an die Zielmoleküle wird durch eine hohe Spezifität und/oder Affinität zu dem Zielmolekül der erfindungsgemäßen Peptide hervorgerufen. Die gebildeten Komplexe haben eine geringe Dissoziationskonstante (KD-Wert).

Mittels des Thioflavin-T-Tests konnte gezeigt werden, dass die erfindungsgemäßen Peptide die Fibrillenbildung von A-Beta-Peptiden sehr effizient hemmen, besonders SEQ ID NO: 1 - 5, insbesondere SEQ ID NO: 1 und/oder SEQ ID NO: 5.

### Beispiele

1. Die in Tabelle 1 aufgeführten D3-Varianten wurden chemisch synthetisiert.

**Tabelle 1: Auflistung der untersuchten D3-Derivate**

| **Bezeichnung des Peptids (mit Erklärung)** | **Aminosäuresequenz (D-enantiomer)** |
|---|---|
| D3 (SEQ ID NO: 11)* | rprtrlhthrnr |
| NT-D3 (D3-N-Terminus) (SEQ ID NO: 6)* | rprtrl |
| RD1 (rational D3) (SEQ ID NO: 3)* | pnhhrrrrrttl |
| RD2 (rational D3) (SEQ ID NO: 2) | ptlhthnrrrrr |
| RD3 (rational D3) (SEQ ID NO: 4)* | rrptlrhthnrr |
| D3Δhth (D3 mit Deletion hth) (SEQ ID NO: 1)* | rprtrlrnr |

| | |
|---|---|
| * nicht erfindungsgemäße Vergleichsbeispiele | |

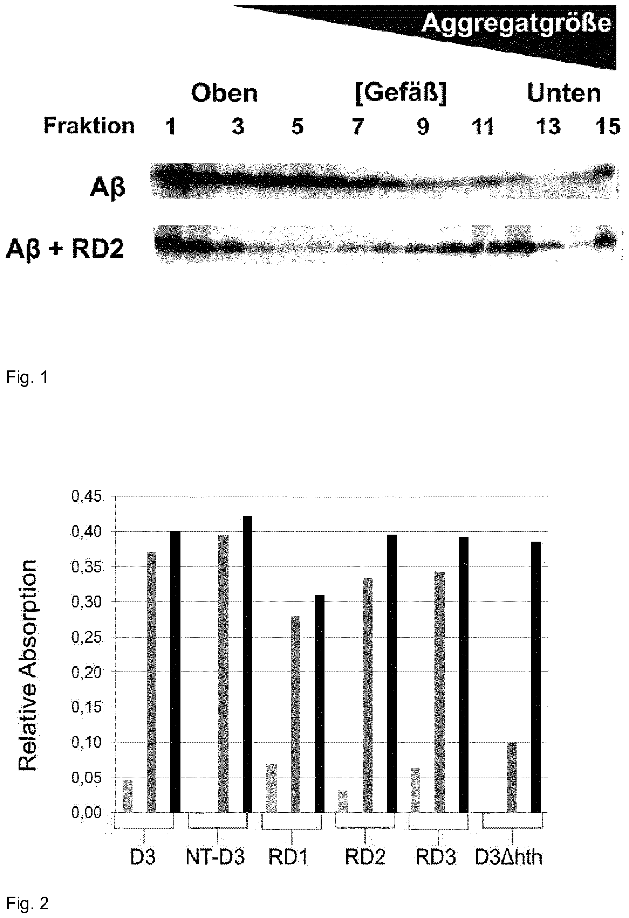

Die Auswirkungen der in der Tabelle beschriebenen Peptide auf die A-Beta-Aggregation wurden mittels Dichtegradientenzentrifugation untersucht. Dabei wurde A-Beta mit dem jeweiligen Peptid vermischt und es erfolgt eine Auftrennung der entstehenden Partikel nach ihrer Größe. Die zu untersuchende Probe wurde auf die Oberfläche des Zentrifugenröhrchens gegeben, in dem ein Dichtegradient vorgelegt wurde, der in diesem Beispiel aus Schichten unterschiedlicher lodixanol-Konzentrationen bestand. Während der mehrstündigen Trennung sedimentieren die Moleküle mit unterschiedlicher Geschwindigkeit im Lösungsmittel, und zwar umso schneller, je größer die Partikel sind. Beendet man die Zentrifugation zu einem geeigneten Zeitpunkt, erhält man in den unterschiedlichen Schichten verschiedene Bestandteile der Probe, die man mittels SDS-PAGE analysiert. Als Kontrolle diente A-Beta ohne Zugabe von Peptid. Beispielhaft ist in Fig. 1 die Auswertung der Läufe von A-Beta und A-Beta mit RD2 gezeigt.

Wie in der Fig.1 ersichtlich, ist in der A-Beta-Probe in allen Fraktionen A-Beta nachzuweisen. Dies ändert sich nach Zugabe von RD2. Hier ist in den Fraktionen 3-9 (A-Beta-Oligomerfraktion) weniger A-Beta-Oligomere nachzuweisen. Es entstehen große Aggregate, die in den Fraktionen 10-15 detektierbar sind. Bei der eingesetzten Peptidkonzentration von 20 µM zeigte D3 keinen Effekt. In früheren Studien stellte sich heraus, dass D3 in höheren Konzentrationen A-Beta-Oligomere präzipitiert und in große, amorphe und untoxische Aggregate umwandelt (Funke et al., ACS Chem. Neurosci. 2010). Alle D-enantiomeren Peptide, die mit A-Beta mit der oben genannten Methode charakterisiert wurden, waren FITC markiert. Die Menge des in Fraktion 12 nachweisbaren Farbstoffs wurde genutzt, im die Bindungsstärke des jeweiligen D3-Derivats an A-Beta-Oligomer bzw. dessen Oligomer-ausfällende Wirkung in *vitro* abzuschätzen. Die Ergebnisse für alle Peptide sind in Tabelle 2 dargestellt. Es zeigte sich, dass vor allem das Peptid RD2 gegenüber D3 eine deutlichere Wirkung auf die A-Beta-Aggregation hat.

**Tabelle 2: Zusammenstellung der Ergebnisse der Modulation des A-Beta1-42-Aggregationsverhaltens verschiedener D3-Derivate**

| **Peptid** | **In Fraktion 12 gebundenes Peptid [%]** |
|---|---|
| A-Beta-Kontrolle ohne Peptid | 0 |
| D3* | 4 |
| NT-D3* | 0 |
| RD1* | 3 |
| RD2 | 20 |
| RD3* | 4 |
| D3Ahth* | 2 |

| | |
|---|---|
| * nicht erfindungsgemäße Vergleichsbeispiele | |

**2.** Die Peptide wurden außerdem bzgl. ihrer *in* vitro-Bindung an verschiedene A-Beta-Konformere (A-Beta-Monomere, -Oligomere, -Fibrillen) im ELISA getestet. Alle Peptide zeigten eine schwache Bindung an A-Beta-Monomere, aber relative hohe Affinitäten zu Oligomeren und Fibrillen (Fig. 2). Interessanterweise wurden am Amino-Terminus biotinylierte Monomere nicht detektiert, während die "seedless"-Monomere (aggregationskeimfrei präpariert) mit der Carboxy-terminalen Biotinylierung schwach gebunden wurden. Dies kann als potentieller Hinweis auf eine am Amino-Terminus lokalisierte Bindeepitopstelle der D3-Derivate interpretiert werden.

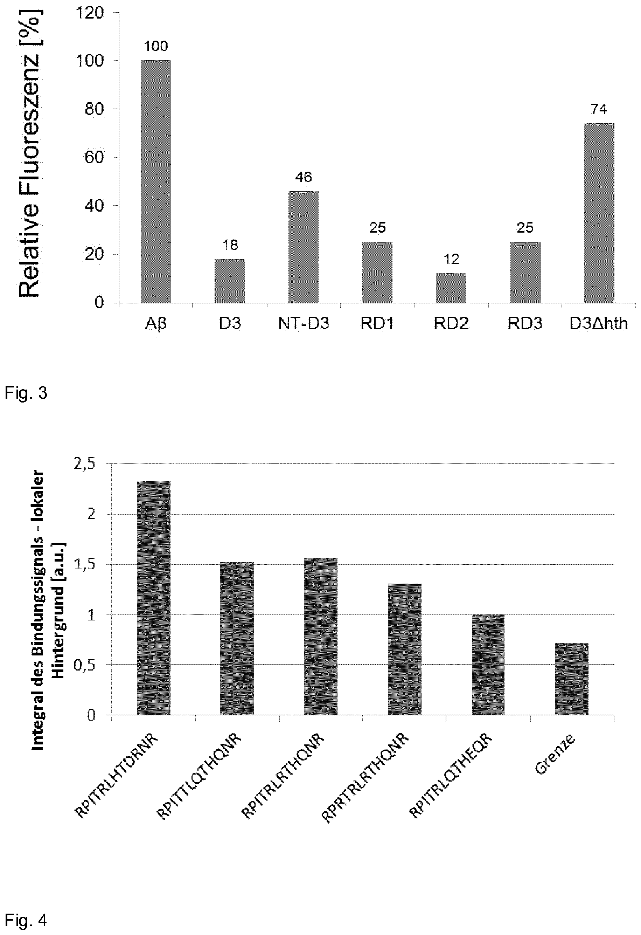

**3.** Einige Peptide wurden im Thioflavin T (ThT)-Aggregationstest eingesetzt. ThT ist ein Fluoreszenzfarbstoff, der an β-Faltblatt-reiche Strukturen verschiedener amyloider Proteine bindet und nach der Anregung bei 440 nm fluoresziert. Die Emission kann auf diese Weise mit dem relativen Fibrillengehalt in der Probe korreliert werden. ThT-Tests werden zur Messung der Fibrillation von A-Beta genutzt und vor allem bei Liganden verwendet, um eine mögliche inhibitorische Wirkung dieser auf die A-Beta-Aggregation nachzuweisen. Die Peptide wurden im Verhältnis 1:10 (A-Beta : Peptid) mit einer 10 µM A-Beta-Lösung eingesetzt. Fünfzehn Stunden später, nach Erreichen der Sättigungsphase in der A-Beta-Kontrolle ohne Zusatz von Liganden, wurde die ThT-Fluoreszenz der Koinkubation von D-Peptiden und A-Beta ausgewertet und prozentual in Abhängigkeit von der A-Beta-Kontrollinkubation dargestellt. Dabei zeigt sich, dass alle eingesetzten Peptide die A-Beta-Fibrillenbildung deutlich reduzieren (Fig. 3).

**4.** Ferner wurden D3-Varianten identifiziert, die im Vergleich zu D3 eine erhöhte Bindungsstärke für A-Beta, insbesondere für A-Beta-Oligomere, bei mindestens gleicher Auswirkung auf dessen Aggregation aufwiesen. Eine stärkere Bindung lässt einen effizienteren therapeutischen Effekt vermuten.

Durch eine PepSpot-Analyse mit einer Membran der Firma JPT (Berlin), auf der mehr als 300 D3-Varianten immobilisiert waren, wurde versucht, Varianten zu identifizieren, die affiner an A-Beta(1-42)-Oligomere binden. D3, D3-Varianten mit variierenden Aminosäuresequenzen (sog. Sättigungsmutagenese - jede Aminosäure wurde durch alle 19 anderen natürlich vorkommenden Aminosäuren in ihrer D-enantiomeren Form ausgetauscht) und Kontrollen waren auf einer Trioxa-Membran carboxy-terminal immobilisiert. Nach der Inkubation mit 5 µM A-Beta(1-42)-Oligomeren für 5 min und Waschschritten wurden die Signale von gebundenem A-Beta durch einen Anti-A-Beta-Antikörper (6E10) nachgewiesen. Die Bindungssignale wurden als Signalintensität/Fläche ausgewertet. Bei der Analyse wurde die Signalintensität des D3-Originals (Mittelwert) mit dem der Derivate verglichen. Die Aminosäureaustausche, die zu einer stärkeren Bindung von A-Beta-Oligomeren führten, sind zusammenfassend in der Tabelle 3 dargestellt.

**Tab. 3: Dargestellt ist in der ersten Zeile fettgedruckt die D-Aminosäuresequenz von D3. In den darunter liegenden Zeilen sind Art und Ort der Aminosäureaustausche aufgeführt, die zu einer Erhöhung der Bindungsstärke an A-Beta-Oligomere führten.**

| **R** | **P** | **R** | **T** | **R** | **L** | **H** | **T** | **H** | **R** | **N** | **R** |
|---|---|---|---|---|---|---|---|---|---|---|---|
| | | I | | T | | P | | D | Q | Q | |
| | | | | | | Q | | | E | D | |
| | | | | | | R | | | | | |
| | | | | | | S | | | | | |

**5.** Um die Ergebnisse zu bestätigen und durch Kombination der in Tab. 4 beschriebenen Aminosäureaustausche neue Varianten mit noch stärkerer Affinität zu A-Beta zu erhalten, wurden weitere D3-Varianten auf Pepchip-Arrays der Firma Pepscan gekoppelt und auf ihre A-Beta Bindungsstärke untersucht. Bei der Variantenauswahl wurden die bereits beschriebenen Ergebnisse der Pepspot-Membranauswertung zu Grunde gelegt. In 6 unabhängigen Versuchen wurde an die D3-Derivate bindendes A-Beta über eine angekoppelte FITC-Markierung nachgewiesen. Dabei wurden 5 µM A-Beta-FITC eingesetzt. Der Nachweis erfolgte in einem Microarrayscanner Typ FLA8000 der Firma Fujifilm, die Bindungssignale wurden mit der AIDA Array Metrix-Software gemessen und mit Hilfe von Mathlab Version 7.10.0.449 ausgewertet. Fig. 4 zeigt die Bindungssignale eines ausgewählten Pepchips als Beispiel.

Fünf der auf den Pepchips immobilisierten Peptide übertrafen in mindestens vier von sechs Versuchen den Grenzwert und zeigten damit im Vergleich zu D3 eine deutlich höhere Bindungsaffinität zu A-Beta. Die Sequenzen dieser Peptide sind in Tab. 4 zusammengefasst. Unter ihnen finden sich Sequenzen mit kombinierten Austauschen aus Tab. 3.

**Tab. 4: Sequenzen der D-Peptide, die im Vergleich zu D3 in immobilisierter Form eine stärkere A-Beta-Monomer-Bindungsstärke aufwiesen**

| **Sequenz** | **Name** |
|---|---|
| RP**I**TRLHT**D**RNR (SEQ ID NO: 7) | DB1 |
| RP**I**T**T**L**Q**TH**Q**NR (SEQ ID NO: 8) | DB2 |
| RP**I**TRL**R**TH**Q**NR (SEQ ID NO: 5) | DB3 |
| RPRTRL**R**TH**Q**NR (SEQ ID NO: 9) | DB4 |
| RP**I**TRL**Q**THE**Q**R (SEQ ID NO: 10) | DB5 |

### Beschreibung der Figuren

Fig. 1: Modulation des A-Beta1-42-Aggregationsverhaltens durch RD2, analysiert mit Hilfe eines lodixanol-Dichtegradienten. Der lodixanolgradient wurde mit 100 µl einer 80 µM A-Beta1-42-Lösung und 100 µl eines 80 µM A-Beta und 20 µM RD2 Gemischs überschichtet. Anschließend wurde 3 h lang bei 4 °C mit 259.000 × g zentrifugiert. Die 15 Fraktionen (die 15. Fraktion ist das mit dem Auftragspuffer aufgekochte Pellet) wurden unmittelbar nach dem Zentrifugationslauf manuell geerntet und über Tris-Tricin-SDS-PAGE mit anschließender Silberfärbung analysiert. Wurde der Lauf unter gleichen Bedingungen mit 20 µM D3 durchgeführt, ergab sich keine Änderung im Vergleich zu dem Lauf nur mit A-Beta.
Fig. 2: ELISA zur relativen Quantifizierung der Bindung der Peptide an verschiedene A-Beta1-42 Konformere. Seedless-Monomere aus Carboxyterminal biotinyliertem A-Beta (hellgraue Balken) und Oligomere (dunkelgraue Balken) und Fibrillen (schwarze Balken) aus Aminoterminal biotinyliertem A-Beta-Monomeren wurden zu je 5 µg/ml immobilisiert und das D-Peptid in einer Konzentration von 10 µg/ml appliziert. Dargestellt ist die relative Quantifizierung der Bindung der Peptide in einer zweifachen Bestimmung als Absorption bei 450 nm nach Abzug der Hintergrundabsorption.
Fig. 3: ThT-Aggregationstest von A-Beta1-42 zur Quantifizierung des relativen Fibrillengehalts in Gegenwart verschiedener Peptide. Die Konzentration von A-Beta betrug 10 µM, die Peptide wurden im Verhältnis von 1:10 (A-Beta : Peptid) hinzugegeben. Die Fluoreszenz von 10 µM A-Beta wurde als 100 % gesetzt und die Werte und Standardabweichungen der übrigen Inkubationen sind als prozentuale Anteile dieses Maximalwerts angegeben. Keines der Peptide zeigte ohne A-Beta eine signifikante ThT-Fluoreszenz.
Fig. 4: Ergebnisse eines Pepchipexperiments. Auf dem Pepchip waren verschiedene D3-Varianten immobilisiert. Der Pepchip wurde mit FITCmarkiertem A-Beta (5 µM) inkubiert. Die FITC-Fluoreszenz-Intensität wurde als Maß für die Bindungsstärke der jeweiligen D3-Derivate ausgelesen. Für die an 11 verschiedenen Positionen auf dem Chip ebenfalls aufgetragenen D3-Kontrollen wurden aus den 11 erhaltenen Werten der Mittelwert und die Standardabweichung berechnet. Mittelwert und Standardabweichung wurden addiert und das Ergebnis wurde als die Grenze definiert, die ein D3-Derivat erreichen muss, um im Vergleich zu D3 eindeutig affiner für A-Beta zu gelten. Gezeigt sind die Integrale der Bindungssignale aller Peptide, die diese Grenze überstiegen. Den einzelnen Balken sind die Mittelwerte dreier exakt gleicher Peptidspots zu Grunde gelegt. a.u.: arbitary units, relative Fluoreszenzeinheiten. Wegen der beobachteten Varianz der Ergebnisse wurde dieser ganze Versuch sechsmal durchgeführt.

## Patentansprüche

1. Peptid, enthaltend mindestens eine Aminosäuresequenz gemäß SEQ ID NO: 2 (RD2:ptlhthnrrrrr).

2. Peptid nach Anspruch 1 **dadurch gekennzeichnet, dass** dies mit einer weiteren Substanz verknüpft ist.

3. Peptid nach einem der vorangehenden Ansprüche enthaltend ein Monomer ausgewählt aus der Gruppe bestehend aus SEQ ID NO: 1, SEQ ID NO: 3, SEQ ID NO: 4, SEQ ID NO: 5, SEQ ID NO: 6, SEQ ID NO: 7, SEQ ID NO: 8, SEQ ID NO: 9 und/oder SEQ ID NO 10.

4. Peptid nach einem der vorangehenden Ansprüche zur Verwendung in vivo zur Bindung an aggregierte Amyloid-Beta-Peptide.

5. Peptid nach einem der Ansprüche 1 bis 3 zur Verwendung in vivo zur Hemmung der Fibrillenbildung von Amyloid-Beta-Peptiden.

6. Peptid nach einem der Ansprüche 1 bis 3 zur Verwendung in vivo zur Verhinderung von Amyloid-Beta-Oligomeren und/oder Amyloid-Beta-Peptidaggregaten.

7. Peptid nach einem der Ansprüche 1-3 zur Verwendung in vivo zur Enttoxifizierung von toxischen Amyloid-Beta-Oligomeren und/oder Aggregaten.

8. Peptid nach einem der Ansprüche 1 bis 3 zur Verwendung in der Medizin.

9. Peptid nach einem der Ansprüche 1 bis 3 zur Verwendung in der Behandlung und/oder Prävention der Morbus Alzheimer.

10. Zusammensetzung enthaltend ein Peptid nach einem der Ansprüche 1-3.

11. Kit, enthaltend ein Peptid nach einem der Ansprüche 1 - 3 ggf. mit/in Puffern oder Lösungen.

12. Verwendung eines Peptid nach einem der Ansprüche 1-3 in vitro als Sonde zur Identifizierung, quantitativen und/oder qualitativen Bestimmung von Amyloid-Beta-Fibrillen und/oder Amyloid-Beta-Oligomeren.

## Claims

1. Peptide containing at least one amino acid sequence according to SEQ ID NO: 2 (RD2:ptlhthnrrrrr).

2. Peptide according to claim 1 **characterized in that** it is linked to a further substance.

3. Peptide according to any one of the preceding claims comprising a monomer selected from the group consisting of SEQ ID NO: 1, SEQ ID NO: 3, SEQ ID NO: 4, SEQ ID NO: 5, SEQ ID NO: 6, SEQ ID NO: 7, SEQ ID NO: 8, SEQ ID NO: 9 and/or SEQ ID NO 10.

4. Peptide according to any one of the preceding claims for use in vivo for binding to aggregated amyloid beta peptides.

5. Peptide according to any one of claims 1 to 3 for use in vivo to inhibit fibril formation of amyloid beta peptides.

6. Peptide according to any one of claims 1 to 3 for use in vivo to prevent amyloid beta oligomers and/or amyloid beta peptide aggregates.

7. Peptide according to any one of claims 1-3 for use in vivo to detoxify toxic amyloid beta oligomers and/or aggregates.

8. Peptide according to any one of claims 1-3 for use in medicine.

9. Peptide according to any one of claims 1 to 3 for use in the treatment and/or prevention of Alzheimer's disease.

10. Composition comprising a peptide according to any one of claims 1-3.

11. Kit containing a peptide according to any one of claims 1-3 optionally with/in buffers or solutions.

12. Use of a peptide according to any one of claims 1-3 in vitro as a probe for the identification, quantitative and/or qualitative determination of amyloid beta fibrils and/or amyloid beta oligomers.

## Revendications

1. Peptide contenant au moins une séquence d'acides aminés selon SEQ ID NO : 2 (RD2 : ptlhthnrrrrr).

2. Peptide selon la revendication 1, **caractérisé en ce qu'**il est lié à une autre substance.

3. Peptide selon l'une des revendications précédentes, contenant un monomère choisi dans le groupe constitué par SEQ ID NO : 1, SEQ ID NO : 3, SEQ ID NO : 4, SEQ ID NO : 5, SEQ ID NO : 6, SEQ ID NO : 7, SEQ ID NO : 8, SEQ ID NO : 9 et/ou SEQ ID NO : 10.

4. Peptide selon l'une des revendications précédentes, destiné à être utilisé in vivo pour se lier à des peptides amyloïdes bêta agrégés.

5. Peptide selon l'une des revendications 1 à 3, destiné à être utilisé in vivo pour inhiber la formation de fibrilles de peptides amyloïdes bêta.

6. Peptide selon l'une des revendications 1 à 3, destiné à être utilisé in vivo pour empêcher la formation d'oligomères amyloïdes bêta et/ou d'agrégats de peptides amyloïdes bêta.

7. Peptide selon l'une des revendications 1 à 3, destiné à être utilisé in vivo pour détoxifier des oligomères et/ou des agrégats amyloïdes bêta toxiques.

8. Peptide selon l'une des revendications 1 à 3, destiné à être utilisé en médecine.

9. Peptide selon l'une des revendications 1 à 3, destiné à être utilisé dans le traitement et/ou la prévention de la maladie d'Alzheimer.

10. Composition contenant un peptide selon l'une des revendications 1 à 3.

11. Kit contenant un peptide selon l'une des revendications 1 à 3, le cas échéant avec/dans des tampons ou des solutions.

12. Utilisation d'un peptide selon l'une des revendications 1 à 3 in vitro en tant que sonde pour l'identification, la détermination quantitative et/ou qualitative de fibrilles amyloïdes bêta et/ou d'oligomères amyloïdes bêta.
